# EUROPÄISCHE PATENTSCHRIFT

(11) **EP 1 173 475 B1**
(45) Veröffentlichungstag und Bekanntmachung des Hinweises auf die Patenterteilung: **31.08.2005**
(21) Anmeldenummer: 00922458.5
(22) Anmeldetag: 03.04.2000
(51) Int. Cl.: C07K 14/025, A61K 47/48

(54) **FRAGMENTE DES VIRUS PROTEINS 2 ODER 3 DES POLYOMAVIRUS ALS FÄHREN FÜR WIRKSTOFFE**
FRAGMENTS OF VIRUS PROTEIN 2 OR 3 OF THE POLYOMA VIRUS, USED FOR TRANSPORTING ACTIVE INGREDIENTS
FRAGMENTS DE LA PROTEINE VIRALE 2 OU 3 DU VIRUS DU POLYOME, SERVANT AU TRANSPORT DE PRINCIPES ACTIFS

(30) Priorität: 10.04.1999 DE 19916224
(43) Veröffentlichungstag der Anmeldung: 23.01.2002
(60) Teilanmeldung: 05009073.7
(73) Patentinhaber: November Aktiengesellschaft Gesellschaft für Molekulare Medizin, 91056 Erlangen (DE)
(72) Erfinder: BERTLING, Wolf, D-91056 Erlangen (DE); REISER, Christian, D-96047 Bamberg (DE); WALTER, Jürgen, D-91056 Erlangen (DE)
(74) Vertreter: Gassner, Wolfgang
(86) Internationale Anmeldenummer: PCT/DE2000/000976
(87) Internationale Veröffentlichungsnummer: WO 2000/061616

(56) Entgegenhaltungen:
- WO-A-94/04686
- WO-A-96/11274
- DE-A- 19 543 553
- X.S. CHENG ET AL.: "Interaction of polyomavirus internal protein VP2 with the major capsid protein VP1 and implications for participation of VP2 in viral entry" EMBO JOURNAL, Bd. 17, Nr. 12, 15. Juni 1998 (1998-06-15), Seiten 3233-3240, XP002147347 EYNSHAM, OXFORD GB in der Anmeldung erwähnt

### SEQUENZPROTOKOLL 1

<110> november AG
<120> Synthetisches Aminosaeuremolekuel
<130> VP2_Minimal_Sequenz
<140>
   <141>
<160> 1
<170> PatentIn Ver. 2.1
<210> 1
   <211> 11
   <212> PRT
   <213> Polyomavirus sp.
<300>
   <302> Sequenz abgeleitet von VP2 Position 287-297
   <303> EMBO J.
   <304> 1998
   <305> 17/12
   <306> 3233-3240
   <308> J02288/EMBL
   <309> 1995-08-22
<400> 1
   Trp Met Leu Pro Leu Ile Leu Gly Leu Tyr Gly
    1               5                  10

### SEQUENZPROTOKOLL 2

<110> november AG
<120> Synthetisches Aminosaeuremolekuel
<130> Bsp. f. VP2 Anker u. Wirkstoff-Peptid
<140>
   <141>
<160> 1
<170> PatentIn Ver. 2.1
<210> 1
   <211> 33
   <212> PRT
   <213> Künstliche Sequenz
<220>
   <223> Beschreibung der künstlichen Sequenz: Pos. 1-21 RT
   aus HIV-1; 22-33 VP2- Sequenz aus Polyoma
<400> 1
   Leu Ala Glu Asn Arg Glu Ile Leu Lys Glu Pro Val His Gly Val Tyr
     1               5                  10                  15
   Tyr Asp Pro Ser Lys Pro Asp Trp Met Leu Pro Leu Ile Leu Gly Leu
                20                  25                  30
   Tyr

### SEQUENZPROTOKOLL 3

<110> november AG
<120> Synthetisches Aminosaeuremolekuel
<130> VP2_Anker Pos. 263-296
<140>
   <141>
<160> 1
<170> PatentIn Ver. 2.1
<210> 1
   <211> 34
   <212> PRT
   <213> Polyomavirus sp.
<300>
   <302> VP2 Anker abgeleitet von der VP2 Sequenz Position 263-296
<303> EMBO J.
   <304> 1998
   <305> 17/12
   <306> 3233-3240
   <308> J02288/EMBL
   <309> 1995-08-22
<400> 1
   Gln Asp Glu Ser Gly Glu Val Ile Lys Phe Tyr Gln Ala Gln Val Val
   1 5 10 15
   Ser His Gln Arg Val Thr Pro Asp Trp Met Leu Pro Leu Ile Leu Gly
   20 25 30
   Leu Tyr

### SEQUENZPROTOKOLL 4

<110> november AG
<120> Synthetisches Aminosaeuremolekuel
<130> VP2_ProtSeq Polyoma sp.
<140>
   <141>
<160> 1
<170> PazenzIn Ver. 2.1
<210> 1
   <211> 319
   <212> PRT
   <213> Polyomavirus sp.
<300>
   <302> VP2 (Kapsid Protein), Genlocus PLY2CG, komplettes
   Genom der Polyoma Staemme a2 und a3 mit der Accession
   Nummer J02288 d. European Molecular Biology Laboratory
   (EMBL, Outstation Eur.Bioinform.Inst.)
<303> EMBO J.
   <304> 1998
   <305> 17/12
   <306> 3233-3240
   <308> J02288/EMBL
   <309> 1995-08-22
<400> 1
   Met Gly Ala Ala Leu Thr Ile Leu Val Asp Leu Ile Glu Gly Leu Ala
   1 5 10 15
   Glu Val Ser Thr Leu Thr Gly Leu Ser Ala Glu Ala Ile Leu Ser Gly
   20 25 30
   Glu Ala Leu Ala Ala Leu Asp Gly Glu Ile Thr Ala Leu Thr Leu Glu
   35 40 45
   Gly Val Met Ser Ser Glu Thr Ala Leu Ala Thr Met Gly Ile Ser Glu
   50 55 60
   Glu Val Tyr Gly Phe Val Ser Thr Val Pro Val Phe Val Ser Arg Thr
   65 70 75 80
   Ala Gly Ala Ile Trp Leu Met Gln Thr Val Gln Gly Ala Ser Thr Ile
   85 90 95
   Ser Leu Gly Ile Gln Arg Tyr Leu His Asn Glu Glu Val Pro Thr Val
   100 105 110
   Asn Arg Asn Met Ala Leu Ile Pro Trp Arg Asp Pro Ala Leu Leu Asp
   115 120 125
   Ile Tyr Phe Pro Gly Val Asn Gln Phe Ala His Ala Leu Asn Val Val
   130 135 140
   His Asp Trp Gly His Gly Leu Leu His Ser Val Gly Arg Tyr Val Trp
   145 150 155 160
   Gln Met Val Val Gln Glu Thr Gln His Arg Leu Glu Gly Ala Val Arg
   165 170 175
   Glu Leu Thr Val Arg Gln Thr His Thr Phe Leu Asp Gly Leu Ala Arg
   180 185 190
   Leu Leu Glu Asn Thr Arg Trp Val Val Ser Asn Ala Pro Gln Ser Ala
   195 200 205
   Ile Asp Ala Ile Asn Arg Gly Ala Ser Ser Ala Ser Ser Gly Tyr Ser
   210 215 220
   Ser Leu Ser Asp Tyr Tyr Arg Gln Leu Gly Leu Asn Pro Pro Gln Arg
   225 230 235 240
   Arg Ala Leu Phe Asn Arg Ile Glu Gly Ser Met Gly Asn Gly Gly Pro
   245 250 255
   Thr Pro Ala Ala His Ile Gln Asp Glu Ser Gly Glu Val Ile Lys Phe
   260 265 270
   Tyr Gln Ala Gln Val Val Ser His Gln Arg Val Thr Pro Asp Trp Met
   275 280 285
   Leu Pro Leu Ile Leu Gly Leu Tyr Gly Asp Ile Thr Pro Thr Trp Ala
   290 295 300
   Thr Val Ile Glu Glu Asp Gly Pro Gln Lys Lys Lys Arg Arg Leu
   305 310 315

### SEQUENZPROTOKOLL 5

<110> november AG
<120> Synthetisches Aminosaeuremolekuel
<130> Wirkstoff 1 RT/Teilseq. aus HIV
<140>
   <141>
<160> 1
<170> PatentIn Ver. 2.1
<210> 1
   <211> 21
   <212> PRT
   <213> Künstliche Sequenz
<220>
   <223> Beschreibung der künstlichen Sequenz: Pos. 1-20 RT
   aus HIV-1 (Accession Nummer AJ006287) und einem
   zusaetzlichen c-terminalen Cys
<400> 1
   Leu Ala Glu Asn Arg Glu Ile Leu Lys Glu Pro Val His Gly Val Tyr
    1               5                  10                  15
   Tyr Asp Pro Ser Cys
               20

### SEQUENZPROTOKOLL 6

<110> november AG
<120> Synthetisches Aminosaeuremolekuel
<130> Wirkstoff 2 GAG/Teilseq. aus HIV
<140>
   <141>
<160> 1
<170> PatentIn Ver. 2.1
<210> 1
   <211> 17
   <212> PRT
   <213> Human immunodeficiency virus type 1
<220>
   <223> Teilsequenz von GAG aus HIV-1 (Accession Nummer
   AJ006287)
<400> 1
   Gly Ser Glu Glu Leu Arg Ser Leu Tyr Asn Thr Val Ala Thr Leu Tyr
    1               5                  10                  15
   Cys

## Beschreibung

Die Erfindung betrifft ein synthetisches biologisch aktives Molekül zur Verwendung als Medikament.

Aus Chen, X.S., Stehle, T., und Harrison, S.C. (1998): Interaction of polyomavirus internal protein VP2 with the major capsid protein VP1 and implications for participation of VP2 in viral entry, The EMBO Journal, Vol. 17, No. 12, pp. 3233-3240 sind die für eine Verankerung von den Virusproteinen VP2 bzw. VP3 des Polyomavirus am Virusprotein 1 verantwortlichen Wechselwirkungen bekannt. Danach erfolgt die Verankerung im Bereich des C-terminalen Endes des VP2 bzw. VP3.

Aus der US 4,950,599 ist es bekannt, dass das Polyomavirus zum Transport von Wirkstoffen in Zellen geeignet ist. Ferner ist es aus der DE 196 18 797 A1 bekannt, dass ein vom Polyomavirus abgeleitetes Kapsomer sich zum Transport von molekularer Substanz in Zellen eignet.

Aus der EP 0 259 149 A2 ist es bekannt, das innere Kapsidprotein VP6 aus Rotavirus als immunologisches Trägermolekül und als Vakzin zur Stimulierung der Immunantwort gegen Rotavirusinfektionen zu verwenden. Dabei werden immunogene Peptide über nicht näher definierte Peptid-Peptidwechselwirkungen an VP6 gebunden. VP6 bildet hier kein strukturiertes Kapsomer, sondern zeigt im Gegenteil einen ausgeprägten strukturellen Polymorphismus. Das VP6 liegt als Monomer oder in oligomerer Form vor. Es können sich aus oligomerem VP6 zwar Partikel bilden. Dabei handelt es sich jedoch nicht um ein Kapsid oder ein Kapsomer, sondern um ein unstrukturiertes Trägerprotein.

In Redmond, M.J. et al. (1991): Rotavirus particels function as immunological carriers for the delivery of peptides from infectious agents and endogenous proteins, Mol. Immunol. 28, 269-278 ist die Verwendung des inneren Kapsidproteins VP6 aus Rotavirus als Transportpartikel bekannt. Dabei ist das VP6 über ein von der Peptidsequenz des rotaviralen Proteins VP4 abgeleitetes Bindeprotein an immunogene Peptide oder Proteine gebunden. Ein an die von VP4 abgeleitete Peptidsequenz gekoppeltes Antigen befindet sich an der Außenseite des Transportpartikels und ist daher vor Abbau nicht geschützt.

In GB 22 57 431 A ist die Verwendung eines chimären Proteins beschrieben, das vom Hüllprotein des Phagen MS-2 abgeleitet ist. Dieses Protein kann Kapside bilden. Daran gekoppelte antigene Peptide oder dgl. sind an die Außenseite des Kapsids gebunden. Bei einer spontanen Assembierung des chimären Proteins während der Expression in E.coli besteht ein hohes Kontaminationsrisiko durch bakterielle DNA oder Proteine.

Aus der DE 43 35 025 A1 ist ein endosomolytisch wirksames virusähnliches Partikel bekannt, das auf seiner äußeren Oberfläche mit membranaktiven Peptiden modifiziert ist. Die Herstellung dieses Partikels ist aufwendig.

Aufgabe der Erfindung ist es, die Nachteile nach dem Stand der Technik zu beseitigen. Es soll insbesondere eine einfache Möglichkeit zur gezielten Assoziation von Wirkstoffen mit VP1 des Polyomavirus angegeben werden.

Diese Aufgabe wird durch die Merkmale des Anspruchs 1 gelöst. Zweckmäßige Ausgestaltungen ergeben sich aus den Merkmalen der Ansprüche 2 bis 7.

In der Beschreibung finden die folgenden Definitionen Anwendung:
Abgeleitete Aminosäuresequenz: Aminosäuresequenz, die gegenüber der Aminosäuresequenz, von der sie abgeleitet ist, unverändert ist oder sich davon durch Aminosäureaustausche, Insertionen oder Deletionen unterscheidet.
C-terminales Ende: Bereich oder Region am C-Terminus.
Synthetisches Molekül: Künstlich hergestelltes Molekül.
Koppeln bzw. Ankoppeln: Kovalentes oder nicht kovalentes Binden. Ein nicht kovalentes Binden kann z.B. über eine Chelat-Bindung erfolgen.
Gentechnik: Technik, die Verfahren zum Einbringen definierter Nukleinsäuren in Zellen umfaßt.

Nach Maßgabe der Erfindung ist ein synthetisches biologisch aktives Molekül vorgesehen, wobei eine vom C-terminalen Ende des Virus Proteins 2 (VP2) bzw. 3 (VP3) des Polyomavirus abgeleitete Aminosäuresequenz (A1) mit einem Wirkstoff verbunden ist.

Das vorgeschlagene synthetische biologisch aktive Molekül ermöglicht auf einfache Weise die gezielte Assoziation von Wirkstoffen mit VP1 des Polyomavirus. Dabei bildet sich ein strukturiertes Kapsomer aus. Unter Verwendung desselben ist eine einfache Herstellung von Kapsiden als universelle Träger für Wirkstoffe möglich.

Vorteilhafterweise besteht die Aminosäuresequenz (A1) aus 10 bis 55, vorzugsweise aus 28 bis 38, Aminosäuren. Die Beschränkung auf eine relativ kurze Aminosäuresequenz vereinfacht und verbilligt die Herstellung des synthetischen biologisch aktiven Moleküls.

Die Aminosäuresequenz entspricht zweckmäßigerweise zumindest abschnittsweise der VP2-Sequenz von der Aminosäureposition 250 bis 319, vorzugsweise von der Aminosäureposition 260 bis 300, besonders vorzugsweise von der Aminosäureposition 287 bis 297. Diese Aminosäuresequenz gewährleistet eine sichere Verankerung am VP1.

Beim synthetischen biologisch aktiven Molekül weist die Aminosäuresequenz (A1) vorzugsweise Aminosäuren in folgender Sequenz auf:

Der Wirkstoff ist vorzugsweise über einen Linker an die Aminosäuresequenz (A1) gebunden. Der Linker kann aus mindestens einer Aminosäure, einem Peptid, Protein, Lipid oder dgl. gebildet sein. Der Wirkstoff kann aus der folgenden Gruppe ausgewählt sein: Nukleinsäure, Oligonukleotid, Protein, Peptid, peptidische Substanz, PNA, Modifikationen der genannten Substanzen und niedermolekulare pharmazeutische Wirkstoffe. Geeignet sind insbesondere solche Wirkstoffe, welche über eine der unten erwähnten reaktiven Gruppen an die Aminosäuresequenz ankoppeln.

Das synthetische biologisch aktive Molekül kann gekoppelt an eine von VP1 des Polyomavirus abgeleitete Aminosäuresequenz vorliegen und/oder Bestandteil eines Medikaments sein.

Zur Herstellung des erfindungsgemäßen synthetischen biologisch aktiven Moleküls eignet sich ein Verfahren mit folgenden Schritten:
a) Bereitstellen einer vom C-terminalen Ende des Virus Proteins 2 (VP2) bzw. 3 (VP3) des Polyomavirus abgeleiteten Aminosäuresequenz (A1), wobei die Aminosäuresequenz (A1) ein Mittel zum Ankoppeln aufweist und
b) Binden des Wirkstoffs über das Mittel zum Ankoppeln an die Aminosäuresequenz (A1).

Das Mittel zum Ankoppeln kann als Aminosäure Glycin, Cystein oder über Lysin gebundenes Glycin aufweisen. Vorteilhafterweise ist das Mittel zum Ankoppeln eine an das N- oder C-terminale Ende der Aminosäuresequenz (A1) gebundene weitere, vorzugsweise synthetisch hergestellte, Aminosäuresequenz (A2).

Das synthetische biologisch aktive Molekül kann, zumindest teilweise, gentechnisch hergestellt werden. Dabei kann die Aminosäuresequenz (A1), die weitere Aminosäuresequenz (A2) und der Wirkstoff ganz oder teilweise gentechnisch hergestellt sein. Die weitere Aminosäuresequenz (A2) weist zweckmäßigerweise Glycine und/oder Aminosäuren mit funktionellen Seitengruppen auf, wobei die funktionellen Seitengruppen aus der folgenden Gruppe ausgewählt sein können: Amino-, Sulfhydryl-, Carboxyl-, Hydroxyl-, Guanidinium-, Phenyl-, Indol-, Imidazol-Rest.

Das Mittel zum Ankoppeln kann eine über eine Aminosäure, vorzugsweise Glycin, Cystein oder über Lysin gebundenes Glycin, an das C- oder N-terminale Ende der Aminosäuresequenz (A1) gebundene reaktive Gruppe sein. Sie kann einen der folgenden Bestandteile aufweisen: Aminosäure mit Monobromacetylrest, Aminosäure mit Monochloracetylrest, Aminosäure mit 3-Nitro-2-Pyridinsulfenylrest (Npys). Die vorgeschlagenen reaktiven Gruppen sind universell einsetzbar. Sie eignen sich zum Ankoppeln einer Vielzahl von Wirkstoffen.

Als besonders vorteilhaft hat es sich erwiesen, dass der Wirkstoff über eine Thioether- oder Disulfidbrücken-Bindung an die Aminosäuresequenz (A1) oder an die weitere Aminosäuresequenz (A2) gebunden wird. Die Herstellung einer solchen Bindung läßt sich in der Praxis einfach bewerkstelligen. Selbstverständlich ist auch die Verwendung anderer reaktiver Gruppen denkbar. Z.B. kommt N-Succinimidyl Bromacetat oder N-Succinimidyl 3-(2-pyridyldithio) -propionat (SPDP) in Betracht.

Der Wirkstoff kann über einen Linker an die Aminosäuresequenz (A1) oder die weitere Aminosäuresequenz (A2) gebunden werden. Der Linker kann aus mindestens einer Aminosäure, einem Peptid, Protein, Lipid oder dgl. gebildet sein.

Zur Herstellung des erfindungsgemäßen synthetischen biologisch aktiven Moleküls eignet sich desweiteren ein Verfahren mit folgenden Schritten:
aa) Synthetisieren einer vom C-terminalen Ende des Virus Proteins 2 (VP2) bzw. 3 (VP3) des Polyomavirus abgeleiteten Aminosäuresequenz (A1) und
bb) Ankoppeln und Synthetisieren eines Wirkstoffs, nämlich eines Peptids, an die Aminosäuresequenz (A1),
wobei die Schritte lit. aa und lit. bb mittels Peptidsynthese oder mittels gentechnischer Methoden durchgeführt werden.

Beim Schritt lit. bb wird die Aminosäuresequenz (A1) um den Wirkstoff verlängert. Die Verlängerung bzw. das Ankoppeln des Wirkstoffs erfolgt durch fortgesetztes Ankoppeln von Aminosäureresten. Dieses Verfahren läßt sich besonders einfach durchführen.

### Beispiele:

### A. Synthese und Aufreinigung der Peptide:

Peptide werden durch simultane multiple Peptidsynthese (Schnorrenberg, G. und Gerhardt, H. (1989) Tetrahedron 45, 7759) an einem Peptidsynthesizerautomaten (Typ: PSSM-8 der Fa. SHIMADZU, Japan) unter Verwendung der 9-Fluorenylmethoxycarbonyl (Fmoc)/tert. Butyl (But)-Strategie nach Sheppard (Atherton, E. und Sheppard, R.C. (1989) "Solid phase peptide synthesis - a practical approach" IRL Press, Oxford) synthetisiert. Die Kopplungen werden mit jeweils 6 Äquivalenten Fmoc-geschützter Aminosäure/1-Hydroxybenzotriazol (HOBt)/12 Äquivalenten n-Methylmorpholin mit 2-(1H-Benzotriazol-1-yl)-1,2,3,3-tetramethyluronium-tetrafluorborat (TBTU) an einem polymeren Trägerharz (Typ: Tentagel S Trityl Harz, RAPP Polymere, Tübingen) bei einer Beladung von 2mmol/g Harz durchgeführt. Die Peptide enthalten C-terminal eine COOH-Gruppe.

Bei der Synthese finden folgende Schutzgruppen Verwendung:Cys (Trt), Arg (Pbf), Ser (But), Thr (But), Tyr (But), Asp (OBut), Glu (OBut), Asn (Trt), Gln (Trt), Lys (Boc), His(Trt), Trp (Boc) mit Trt: Trityl-, But: t-Butyl, OBut: t-Butylester, Boc: t-Butyloxycarbonyl und Pbf: 2,2,4,6,7-Pentamethyldihydrobenzofuran-5-sulfonyl.

Die Abspaltung aller Schutzgruppen wird mit Trifluoressigsäure (TFA)/Thioanisol/Thiokresol (95:2,5:2,5) in 3 h bei Raumtemperatur unter Zusatz von 3% Triisopropylsilan und anschließender Zugabe von 10% Trimethylchlorsilan für 1 h durchgeführt. Die Peptide liegen nach der Lyophilisation in Form ihrer Trifluoressigsäure-Salze vor.

Die Peptide werden mittels präparativer HPLC an einer Bischoff Polyencap 300 Trennsäule, 10µm, 250x16 mm unter Verwendung eines Gradienten von 0,05 % Trifluoressigsäure in Wasser (Laufmittel A) nach 0,05 % Trifluoressigsäure in 80 % Acetonitril/Wasser (Laufmittel B) aufgereinigt.

Alternativ wurde eine Vydac Trennsäule vom Typ 218 TP 101522 (10-15 µm, 250x22 mm) mit einem Gradienten von 43-73 % Laufmittel B in 30 min bei einem Fluß von 15 ml/min verwendet.

Mittels Peptid-Synthese wird z.B. die folgende Aminosäuresequenz synthetisiert:

An diese Sequenz wird die reaktive Gruppe über eine Aminosäure, vorzugsweise über Glycin, an das N-terminale Ende der Aminosäuresequenz gebunden. Die reaktive Gruppe kann aus Monochloracetyl-Glycin gebildet sein. Alternativ ist es auch möglich, einen Monobromacetylrest anzukoppeln.

### Kopplung von Monochloracetylglycin in der Festphasensynthese:

Gleiche Äquivalente Monochloracetylglycin und 1-Hydroxybenzotriazol (HOBt) werden in Dimethylformamid (DMF) gelöst, mit dem gleichen Äquivalent N,N'-Diisopropylcarbodiimid (DIC) gemischt und zu dem Peptidharz gegeben. Monochloracetylglycin liegt dabei im Vergleich zum beladenen Peptidharz im Überschuß vor. Die Reaktion erfolgt unter gelegentlichem Rühren und sollte mindestens 1 h andauern.

Die reaktive Gruppe ermöglicht eine kovalente Bindung des so hergestellten biologisch aktives Moleküls mit einem Wirkstoff, z.B. einem Peptid, das eine freie SH-Gruppe aufweist. Durch die Reaktion der SH-Gruppe mit dem Chloratom der Monochloracetyl-Gruppe bildet sich eine stabile Thioether Verbindung gemäß der folgenden Reaktionsgleichung:

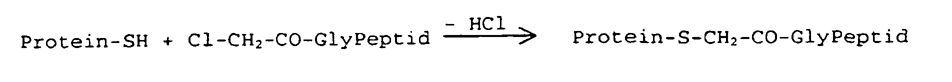

### Konjugatbildung zwischen Monochloracetyl-modifiziertem Ankerpeptid und Peptid mit endständigem Cystein:

Monochloracetyl-modifiziertes Ankerpeptid wird im Überschuß gegenüber dem zu konjugierenden Peptid eingesetzt. Die Reaktion erfolgt in 0,1 M NaHCO₃ zwischen pH 7-8 bei RT. Bei geringer Löslichkeit des Peptids oder des Ankers in wäßriger Lösung wird die Konjugatbildung in 4M Guanidinhydrochlorid, pH 8.0 durchgeführt (Lindner, W. und Robey, F.A. (1987) Int. J. Pept. Protein Res. 30, 794-800). Alternativ läßt sich der organische Lösungsmittelanteil, z.B. DMSO im Reaktionsansatz erhöhen. Zur Vermeidung unerwünschter Nebenprodukte können wasserlösliche Phosphine als Reduktionsmittel zugegegben werden.

Wahlweise läßt sich die Konjugationsreaktion auch unter folgenden Bedingungen durchführen. Das monochloracetylierte Ankerpeptid und ein Peptid mit endständiger SH-Gruppe werden in 1-Methyl-2-pyrrolidon in Gegenwart eines etwa 10-fachen Überschusses an Diisopropylethylamin und eines ca. 5-fachen Überschusses Tributylphosphin bei RT inkubiert. Nach der Reaktion wird H₂O zugefügt und das Produkt durch Zugabe von Ether präzipitiert und durch Gelfiltration aufgereinigt (Defoort, J.P., Nardelli, B., Huang, W. und Tam, J.P.(1992) Int. J. Protein Res. 40, 214-221).

Wahlweise läßt sich die Konjugationsreaktion auch folgendermaßen durchführen. Das die SH-Gruppe enthaltende Peptid wird in 0.2 M Phosphatpuffer, 10 mM EDTA, pH 7.4 gelöst. Hierzu wird das in Dimethylformamid gelöste Monochloracetylmodifizierte Ankerpeptid gegeben. Nach der Reaktion erfolgt die Aufreinigung durch Gelfiltration oder RP-HPLC (Zhang, L. und Tam, J.P. (1997) J. Am. Chem. Soc. 119, 2363-2370).

Zur Herstellung von isolierten VP1 Pentameren wird VP1 in E.coli als rekombinantes Protein mit einem N-terminalen 6 x Histidin Affinitäts-Tag (=His-Tag) exprimiert. Das Protein wird über eine Ni-NTA Affinitätschromatographie gereinigt.

Der His-Tag wird durch eine Faktor Xa-Behandlung entfernt. Das Protein wird in einem SDS-PAGE-Gel mit anschließender Coomassie-Färbung analysiert.

Dieses Ausgangsmaterial (VP1-Protein in 20mM Hepes, pH 7,3, 1 mM EDTA, 200 mM NaCl, 5 % Glycerin) wird in centricon 100 (Amincon) konzentriert und über FPLC-Gelfiltration (Superdex 200) mit einem Laufpuffer (50 mM Tris, 0,15 M NaCl, 5 mM EDTA, pH 8,5) in die hochmolekulare Kapsidfraktion und die Pentamer-Untereinheiten (Molekulargewicht: etwa 225 kD) getrennt. Beide Fraktionen werden in centricon 100 konzentriert. Iodacetamid (SIGMA) wird in einem 10-fach molaren Überschuss zu der die Pentamere enthaltenden Lösung gegeben, um potentiell reaktive SH-Gruppen zu blockieren. Die Reaktion erfolgt für 2 Stunden bei Raumtemperatur. Die modifizierte Pentamerfraktion wird über Gel-Filtration von überschüssigem Iodacetamid getrennt. Mit Hilfe von VP1 spezifischen Antikörpern und einer Affinitätsmatrix (Protein A Support der Firma BIO-RAD) werden VP1-spezifische monoklonale Antikörper adsorbiert. Mit der Antikörper-beschichteten Matrix wird die gereinigte Pentamerfraktion präzipitiert. In einem weiteren Inkubationsschritt wird die Pentamer-Matrix mit der Ankersequenz versetzt. Die Proben werden in einem SDS-Polyacrylamid-Gel (12.5%) analysiert.

### Konjugatbildung zwischen Npys-modifiziertem Anker-Peptid und Peptid mit endständigem Cystein:

Wahlweise kann neben der Reaktion zwischen einem monochlor- oder monobromacetyliertem Ankerpeptid und einem Peptid mit endständigem Cystein unter Ausbildung eines Thioethers die Konjugatbildung zwischen Ankerpeptid und Peptidsequenz auch über die 3-Nitro-2-pyridinsulfenyl-(=Npys) Gruppe an einem endständigen Cystein des Ankers und einer SH-Gruppe des zu koppelnden Peptids erfolgen. Statt eines monochloracetylierten Glycin wird hierzu ein Npys-modifiziertes Cystein an die Ankersequenz N-terminal gekoppelt. Dabei handelt es sich um ein sogenanntes aktiviertes Disulfid, welches in der Lage ist mit Thiolen, wie z.B. Cysteinen, unter Bildung eines unsymmetrischen Disulfids zu reagieren. Dabei kommt es zur Abspaltung von 3-Nitro-2-thiopyridon, dessen UV Maximum bei 329 nm eine spektroskopische Untersuchung der Kinetik der Reaktion zwischen beiden Verbindungen gestattet.

Für die Reaktion werden folgende Bedingungen gewählt (Albericio, F., Andreu, D., Giralt, E., Navalpotro, C., Pedroso, E., Ponsati, B. und Ruiz-Gayo, M. (1989) Int. J. Peptide Res. 34, 124-128). Das in 0.1 M Natriumacetat, 0.1 M Natriumchlorid, pH 4.5 gelöste zu koppelnde Peptid mit endständiger SH-Gruppe wird mit dem Npys-modifizierten Peptid versetzt, anschließend der pH auf 5.0 eingestellt und für mindestens 12 h unter Rühren inkubiert. Danach wird der pH durch Zugabe von 1N NaOH auf 7.0 eingestellt und nochmals 3 h inkubiert. Nach der Reaktion wird der Ansatz gegen 10 mM NaHCO₃ dialysiert.

Die Reaktion läuft optimal in einem pH Bereich zwischen 4.5 und 7.0 ab. Diese Bedingungen gewährleisten eine Minimierung unerwünschter Nebenreaktionen, wie z.B. die Ausbildung von symmetrischen Disulfiden zwischen den zu koppelnden Peptidmolekülen oder eine Abspaltung der Npys-Gruppe. Das Npysmodifizierte Peptid sollte bei der Reaktion im Überschuß gegenüber dem zu konjugierenden Peptid vorliegen (Albericio, F., Andreu, D., Giralt, E., Navalpotro, C., Pedroso, E., Ponsati, B. und Ruiz-Gayo, M. (1989) Int. J. Peptide Res. 34, 124-128).

Beispiele der Erfindung sind in den folgenden Sequenzprotokollen dargestellt:
Das Sequenzprotokoll 1 zeigt eine von VP2 des Polyomavirus, Position 287-297, abgeleitete Aminosäuresequenz. Sie dient im synthetischen biologisch aktiven Molekül als Anker zum Verankern des Wirkstoffs am VP1.
Das Sequenzprotokoll 2 zeigt ein erstes Ausführungsbeispiel eines synthetischen biologisch aktiven Moleküls. Die vom HIV-1 abgeleitete Peptidsequenz entspricht den Positionen 1-21; die als Anker wirkende angekoppelte Aminsosäuresequenz besetzt die Positionen 22-33. Sie ist vom VP2 des Polyomavirus abgeleitet.

Im Sequenzprotokoll 3 ist ein weiteres Beispiel für eine als Anker geeignete Aminosäuresequenz gezeigt.

Das Sequenzprotokoll 4 zeigt die Sequenz des VP2 vom Polyomavirus. Daraus sind die als Anker geeigneten Sequenzen zwischen den Positionen 250 und 300 ersichtlich.

In den Sequenzprotokollen 5 und 6 sind weitere synthetische biologisch aktive Moleküle gezeigt. Sie können mit VP1 des Polyomavirus gekoppelt und anschließend zur Behandlung einer HIV-Infektion in die infizierten Zellen eingeschleust werden.

## Patentansprüche

1. Synthetisches biologisch aktives Molekül, gebildet aus einem Wirkstoff und einer vom C-terminalen Ende des Virus Proteins 2 (VP2) bzw. 3 (VP3) des Polyomavirus abgeleiteten Aminosäuresequenz (A1), wobei an die Aminosäuresequenz (A1) der Wirkstoff gebunden ist, so dass der Wirkstoff mittels der Aminosäuresequenz (A1) mit dem Virus Protein 1 (VP1) des Polyomavirus unter Ausbildung eines strukturierten Kapsomers assoziierbar ist, zur Verwendung als Medikament.

2. Synthetisches biologisch aktives Molekül zur Verwendung als Medikament nach Anspruch 1, wobei die Aminosäuresequenz (A1) aus 10 bis 55, vorzugsweise aus 28 bis 38, Aminosäuren besteht.

3. Synthetisches biologisch aktives Molekül zur Verwendung als Medikament nach einem der vorhergehenden Ansprüche, wobei die Aminosäuresequenz (A1) zumindest abschnittsweise der VP2-Sequenz von der Aminosäureposition 250 bis 319, vorzugsweise von der Aminosäureposition 260 bis 300, besonders vorzugsweise von der Aminosäureposition 287 bis 297, entspricht.

4. Synthetisches biologisch aktives Molekül zur Verwendung als Medikament nach einem der vorhergehenden Ansprüche, wobei die Aminosäuresequenz (A1) Aminosäuren in folgender Sequenz aufweist:

5. Synthetisches biologisch aktives Molekül zur Verwendung als Medikament nach einem der vorhergehenden Ansprüche, wobei der Wirkstoff über einen Linker an die Aminosäuresequenz (A1) gebunden ist.

6. Synthetisches biologisch aktives Molekül zur Verwendung als Medikament nach einem der vorhergehenden Ansprüche, wobei der Wirkstoff aus der folgenden Gruppe ausgewählt ist: Nukleinsäure, Oligonukleotid, Protein, Peptid, peptidische Substanz, PNA, Modifikationen der genannten Substanzen und niedermolekulare pharmazeutische Wirkstoffe.

7. Synthetisches biologisch aktives Molekül zur Verwendung als Medikament nach einem der vorhergehenden Ansprüche, gekoppelt an eine von VP1 des Polyomavirus abgeleitete Aminosäuresequenz.

## Claims

1. A synthetic biologically active molecule composed of an active substance and an amino acid sequence (A1) derived from the C-terminal end of virus protein 2 (VP2) or 3 (VP3) of polyoma virus, wherein the active substance is bound to the amino acid sequence (A1) so that the active substance can be associated with the virus protein 1 (VP1) of polyoma virus by means of the amino acid sequence (A1) with the formation of a structured capsomer, for use as a medicament.

2. A synthetic biologically active molecule for use as a medicament as claimed in claim 1, wherein the amino acid sequence (A1) comprises from 10 to 55, preferably from 28 to 38, amino acids.

3. A synthetic biologically active molecule for use as a medicament as claimed in any of the preceding claims, wherein the amino acid sequence (A1) at least in some sections corresponds to the VP2 sequence from amino acid position 250 to 319, preferably from amino acid position 260 to 300 and particularly preferably from amino acid position 287 to 297.

4. A synthetic biologically active molecule for use as a medicament as claimed in any of the preceding claims, wherein the amino acid sequence (A1) has amino acids in the sequence below:

5. A synthetic biologically active molecule for use as a medicament as claimed in any of the preceding claims, wherein the active substance is bound to the amino acid sequence (A1) via a linker.

6. A synthetic biologically active molecule for use as a medicament as claimed in any of the preceding claims, wherein the active substance is selected from the following group: nucleic acid, oligonucleotide, protein, peptide, peptidic substance, PNA, modifications of said substances and low-molecular-weight pharmaceutically active substances.

7. A synthetic biologically active molecule for use as a medicament as claimed in any of the preceding claims, coupled to an amino acid sequence derived from polyoma virus VP1.

## Revendications

1. Molécule synthétique biologiquement active, formée à partir d'un principe actif et d'une séquence d'acides aminés (A1) dérivée de l'extrémité C-terminale de la protéine virale 2 (VP2) ou 3 (VP3) du polyomavirus ou virus du polyome, dans laquelle le principe actif est lié à la séquence d'acides aminés (A1) de façon telle que le principe actif peut être associé au moyen de la séquence d'acides aminés (A1) à la protéine virale 1 (VP1) du polyomavirus en formant un capsomère structuré, pour une utilisation comme médicament.

2. Molécule synthétique biologiquement active pour une utilisation comme médicament selon la revendication 1, dans laquelle la séquence d'acides aminés (A1) se compose de 10 à 55, de préférence de 28 à 38, acides aminés.

3. Molécule synthétique biologiquement active pour une utilisation comme médicament selon l'une des revendications précédentes, dans laquelle la séquence d'acides aminés (A1)correspond au moins par segments à la séquence VP2 de la position d'acides aminés 250 à 319, de préférence de la position d'acides aminés 260 à 300, de façon particulièrement préférée de la position d'acides aminés 287 à 297.

4. Molécule synthétique biologiquement active pour une utilisation comme médicament selon l'une des revendications précédentes, dans laquelle la séquence d'acides aminés (A1) présente des acides aminés selon la séquence suivante :

5. Molécule synthétique biologiquement active pour une utilisation comme médicament selon l'une des revendications précédentes, dans laquelle le principe actif est lié à la séquence d'acides aminés (A1) par un liant.

6. Molécule synthétique biologiquement active pour une utilisation comme médicament selon l'une des revendications précédentes, dans laquelle le principe actif est sélectionné dans le groupe formé par : acide nucléique, oligonucléotide, protéine, peptide, substance peptidique, ANP, modifications des substances citées et principes actifs pharmaceutiques ayant une faible masse moléculaire.

7. Molécule synthétique biologiquement active pour une utilisation comme médicament selon l'une des revendications précédentes, couplée à une séquence d'acides aminés dérivée de la VP1 du polyomavirus.
